# EUROPEAN PATENT SPECIFICATION

(11) **EP 3 210 013 B1**
(45) Date of publication and mention of the grant of the patent: **11.07.2018**
(21) Application number: 15793905.9
(22) Date of filing: 16.09.2015
(51) Int. Cl.: G01N 33/483, G01N 33/00

(54) **DEVICE FOR PRELIMINARY SCREENING OF ADENOMA OF THE COLON-RECTUM**
VORRICHTUNG ZUM VOR-SCREENING AUF ADENOM DES DICKDARMS-ENDDARMS
DISPOSITIF POUR UN DÉPISTAGE PRÉLIMINAIRE D'UN ADÉNOME DU CÔLON-RECTUM

(30) Priority: 20.10.2014 IT RM20140595
(43) Date of publication of application: 30.08.2017
(73) Proprietor: Scent S.r.l., Ferrara (IT)
(72) Inventor: MALAGÙ, Cesare, I-44121 Ferrara (IT); GIBERTI, Alessio, I-44124 Ferrara (IT); GHERARDI, Sandro, I-44126 Bosco Mesola (FE) (IT); GAIARDO, Andrea, I-38051 Borgo Valsugana (TN) (IT); LANDINI, Nicolò, I-40015 Galliera (BO) (IT); ZONTA, Giulia, I-45030 Castelnovo Bariano (RO) (IT)
(74) Representative: Papa, Elisabetta
(86) International application number: PCT/IB2015/057117
(87) International publication number: WO 2016/063148

(56) References cited:
- WO-A1-2012/083432
- WO-A2-2011/061308
- US-A- 4 584 867
- US-A1- 2011 239 792
- US-B1- 6 423 120
- TIM G. DE MEIJ ET AL: "Electronic nose can discriminate colorectal carcinoma and advanced adenomas by fecal volatile biomarker analysis: proof of principle study", INTERNATIONAL JOURNAL OF CANCER, vol. 134, no. 5, 2 September 2013 (2013-09-02), pages 1132-1138, XP055168060, ISSN: 0020-7136, DOI: 10.1002/ijc.28446 cited in the application
- BHATTACHARYYA N ET AL: "Incremental PNN classifier for a versatile electronic nose", SENSING TECHNOLOGY, 2008. ICST 2008. 3RD INTERNATIONAL CONFERENCE ON, IEEE, PISCATAWAY, NJ, USA, 30 November 2008 (2008-11-30), pages 242-247, XP031410299, ISBN: 978-1-4244-2176-3 cited in the application
- CESARE MALAGÙ ET AL: "Chemoresistive Gas Sensors for the Detection of Colorectal Cancer Biomarkers", SENSORS, vol. 14, no. 10, 1 January 2014 (2014-01-01), pages 18982-18992, XP055168058, DOI: 10.3390/s141018982 cited in the application

## Description

### Technical field of the invention

The present invention relates to a device for screening of cancer/adenoma of the colon-rectum, based on the analysis of volatile organic compounds (*Volatile Organic Compounds, VOCs*) contained in faecal gases.

### Background

The literature describes a project of a device used for the detection of colorectal cancer by fecal gas analysis (TG de Meij et al., "Electronic nose can discriminate colorectal cancer and advanced adenomas by fecal volatile biomarker analysis: proof of principle study ", International Journal of Cancer 134, 1132-1138, 2014). Such device is a so-called "electronic nose" trained by means of neural networks and suitable for analysis of feces previously chilled to -20°C. However, this device is suitable for relative measures rather than absolute. For this reason, and also in consideration of its structural and operational complexity, it is not suitable for employing for routine screening of large populations and also in outpatient setting. Furthermore, it was not originally designed for diagnostic purposes, but it can also be used in other contexts, thus it is not specific.

Preliminary research carried out by our research team, whose results are published in the article CESARE MALAGÙ ET AL: "Chemoresistive Gas Sensors for the Detection of Colorectal Cancer Biomarkers", SENSORS, vol. 14, no. 10, 1 January 2014 (2014-01-01), pages 18982-18992, DOI: 10.3390/s141018982 relates to the analysis of some organic compounds commercially available from gas bottles or originally in liquid form, that have been identified in the literature as indicators of cancer of the colon-rectum and which may be present in gaseous form within the intestine. The measurement equipment includes flowmeters, a computer for collection and processing unit of the real-time response, a cylindrical chamber hermetically closed in which eight chemoresistive sensors are circularly arranged and simultaneously invested by flow of air and gas, transported via pipes made of Teflon. The entire instrumental apparatus is contained in a laboratory, it is not portable and not applicable to the measurement of organic samples such as feces, thus not usable for diagnostic purposes.

In the literature is provided an apparatus, described in the article BHATTACHARYYA N ET AL: "Incremental PNN classifier for a versatile electronic nose", 3RD INTERNATIONAL CONFERENCE ON SENSING TECHNOLOGY, IEEE, PISCATAWAY, NJ, USA, 30 November 2008 (2008-11-30), pages 242-247, ISBN: 978-1-4244-2176-3, however it is an electronic nose that takes advantage of the training of neural networks, used for analysis of the quality of tea. The measurement method is also different from the device object of the patent application, since it requires a preliminary step of "headspace generation", which increases the pressure within the container by means of a valve system, before it can send the organic compounds produced from the tea mixture to the sensitive unit, in which the sensors are arranged in parallel and not in series.

Document WO2011/061308 A2 discloses a detection device for determining colorectal cancer based on volatile organic compounds released from a faeces sample and separated by gas chromatography. A plurality of metal oxide sensors is arranged in series. Document WO2012/083432 A1 discloses a detection device for volatile organic compounds comprising a plurality of metal-oxide semiconductor sensors, each housed in an isolation cell for avoiding interferences.

Document US4584867 A discloses a gas sensor comprising a plurality of different gas sensors arranged in series. A laminar flow of gas is created by a pump. Other systems for the analysis of organic compounds in gaseous form, provided in the literature, are of chromatographic-gas type (US2011/0239792 A1, US6423120 B1) therefore, require a pre-treatment of gas before it is conveyed towards a detector.

The main screening test for colorectal adenomas employed to now is the detection of occult blood in the faeces, generally referred to by the acronym SOF. However, this test - even in its different variants of implementation - is not very sensitive, and its reliability is not satisfactory.

In fact, the JRC (Cancer Colon-Rectum) or adenoma are not revealed if not bleeding at the time of sampling or if they are located in the right region of the colon. In particular, it is estimated that the number of false negatives produced by the SOF test is on average around 20%, thus very high. This brings the patient with adenoma not perform further tests, such as colonoscopy, resulting in belated intervention and increase in the death rate. For this reason, namely in order to increase the sensitivity of the test, it should be recommended to perform the taking of the sample for three consecutive days, dilating time, cost and inconvenience to the patient.
At the same time, the sample may contain blood relating to causes different by the presence of a tumor or adenoma (bleeding from peptic ulcers, angiodysplasia of colon, sickle cell anemia, etc.), thus giving rise to false positives in the analysis.

### Summary

The posed technical problem is solved by the present invention: a device for detection of VOC in the gases faecal that allows to obviate the above mentioned drawbacks with reference to the known art.
This problem is solved by a device according to claim 1 and a method according to claim 5. Preferred features of the present invention are object of the dependent claims.
The invention provides a device for detecting VOC in faecal gases, specifically suitable for screening colorectal cancer/adenomas. The device is based on a sequence of sensors or transducers, preferably in a solid status, and means for generating a laminar flow of air, that laps first the feces sample and then, in series, the sensors / transducers. The sensors are of chemoresistive type. The device comprises a pneumatic system configured to supply a flow of air and an array of gas sensors, preferably in a solid state, capable of revealing the VOC biomarkers of the aforesaid pathology issued by a faeces sample. That measure, analyzed by a specific algorithm, provides the negative or positive result of the diagnosis.

The device is constructionally and functionally simple and reliable, can be portable and can be realized at low cost. The test allowed by the device is performed in vitro and is therefore not invasive, because at the time of the execution of the analysis is not necessary the presence of the patient.
The device thus allows a pre-analysis for diagnosis of colorectal cancer (CRC) since the stage of adenoma.
In particular, the device is suitable for using for the screening of large populations aimed to the identification of cancers or adenomas of the colon-rectum and it is specifically compatible with standardized methods of faeces collection. The collection of faeces samples can be performed by the same patient which, for example, asking to the doctor, will receive a special airtight container, without complicating the procedure currently used for testing SOF.
The device allows to get the test results quickly.
Furthermore, the device has the ability to drastically reduce the number of false negative results of tests performed with methodology SOF, and is suitable to be applied as an additional test to the current ones. The device allows to reduce costs for the care of patients in advanced stages of cancer (such as surgery, chemotherapy drugs, hospitalization) not promptly diagnosed because of false negative provided by the SOF test.

Other advantages, features and modes of employ of the present invention will become evident from the following detailed description of some embodiments thereof, given by way of example and not of limitation.

### Brief description of the drawings

Reference will be made to the figures of the accompanying drawings, in which:
- Figure 1 shows a schematic illustration of a preferred embodiment of the analysis device according to the present invention;
- Figure 2 schematically shows an exemplary structure of the device of Figure 1; and
- Figure 3 shows a perspective view and a longitudinal sectional view of a main container suitable for housing a secondary container of a faeces sample according to a variant of the preferred embodiment of the invention.

### Detailed description of preferred embodiments

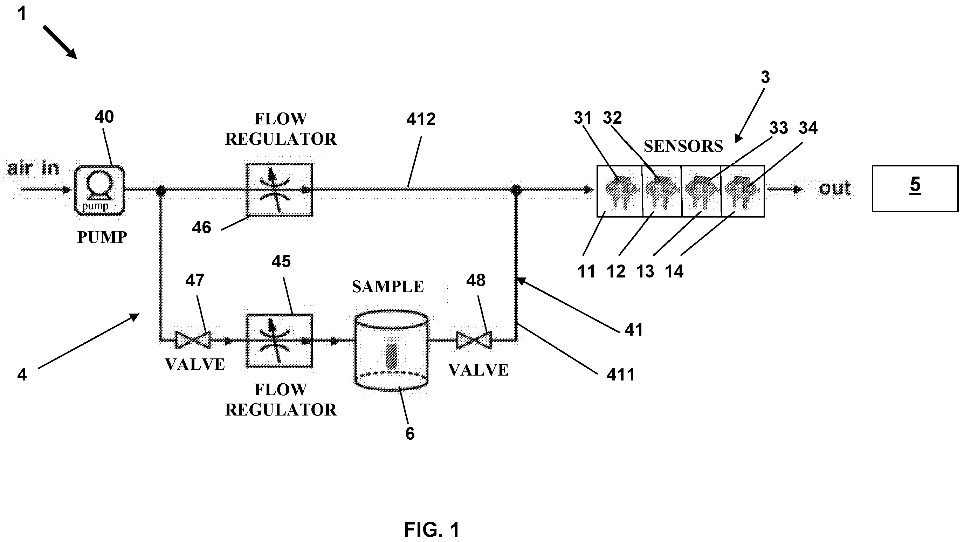

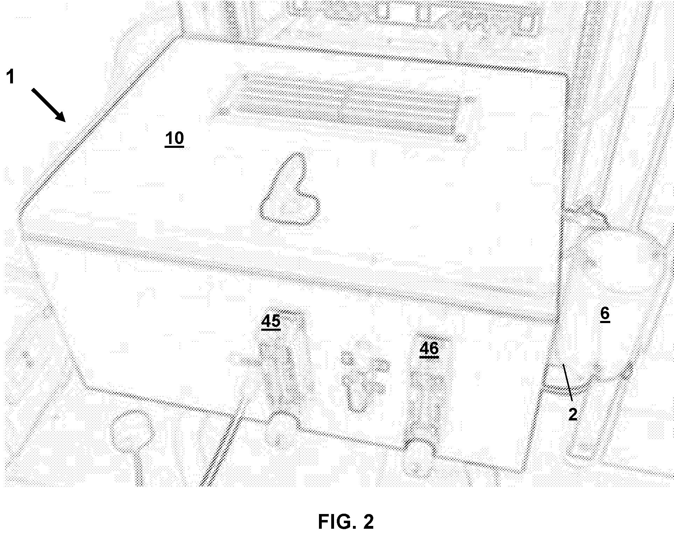

With reference to Figures 1 and 2, a device for detection of VOC in the faecal gases according to a preferred embodiment of the invention is generally denoted by 1.
The device 1 is configured for screening colorectal cancer / adenoma.
The faecal gases are emitted from a faeces sample received in a container 6. Said container may be part of device 1, such as fixed or detachable component of it, or be separately supplied. In any case, the container 6 can be of the disposable type.
The device 1 comprises an outer casing 10 which houses further components. In the present example, from the external walls of the casing means 2 for removable connection with the container 6 spreads, which allows the support to be part of the enclosure 10. Said connection means preferably comprises a pair of flanges or jaws configured to engage the container 6. Regardless of the specific implementation, the connecting means realizes a reception seat of the faeces sample as such or as housed in a main or secondary container.

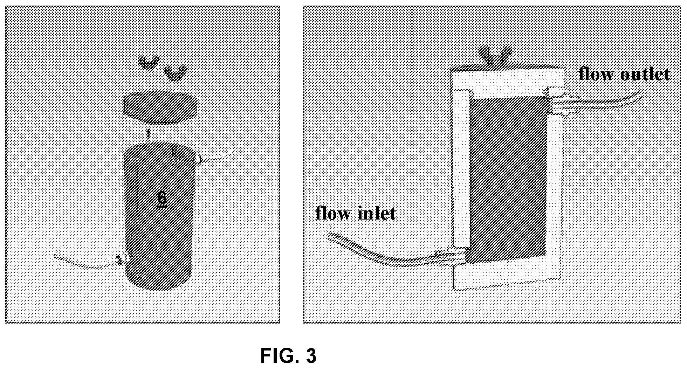

As shown in Figure 3, an alternative embodiment provides a main container apt to receive a secondary container, such as a test tube of known type, which directly accommodates the faeces sample. Such a main container, or sample-holder box, is configured such as to be able to contain test tubes of standard type for the analysis of faeces and simultaneously limit the volume, then time of permanance in order to speed up the measurement. In addition, the air inlet and outlet are positioned in such a way that the flow of air collects the gas exhaled from the faeces sample, and respectively arranged at a bottom and at a top portion of the container.

The air entering the sample holder is piped in parallel with the respect to the air entering in the sensor chamber, and downstream of the container the two flows are mixed so that the exhalations of the sample are diluted and do not bring the sensor to saturation.

The device 1 then comprises a plurality of sensors or transducers, generally denoted by 3, received within the housing 10. In the present example, four sensors have been represented, denoted respectively by the references 31 to 34.

Also in the present example, the casing 10 includes a plurality of chambers 11-14, one for each sensor or transducer. The arrangement is such that the chambers, and then the sensors or transducers housed in them, are traversed in series by a flow of air which will be discussed shortly.

Each sensor or transducer is configured for the detection of one or more respective and specific VOC. In other words, preferably each sensor or transducer is selective to different fractions of the VOCs mixture of interest.

The sensors or transducers 3 are of chemoresistive type, that is capable to change its electrical resistance as a function of surface chemical reactions with a specific VOC or mixture VOCs. In particular, the gas to be monitored, coming into contact with an element/surface layer of the sensor (for example a metal oxide), chemically reacts with it by changing the electrical resistance of the sensor itself. Each change in resistance thus indicates a variation in the concentration of the gaseous species.

In another embodiment, each sensor comprises a substrate of alumina coated by a film of chemoresistive material, the latter preferably deposited by screen printing technique.

Each sensor or transducer can be inserted inside a dedicated electronic circuit to be able to measure the resistivity.

In the present embodiment, the sensors used can be based on semiconductor nanostructures and synthesized either on single metal ion or on more different ions for synthesis. Examples of semiconductor materials which can be used are: compounds of tin and titanium at different concentrations (SnxTiyO2), tin oxide-titanium-niobium (SnNbTiO2), tungsten oxide (WO3), tungsten compounds and tin (WSnOx), zinc oxide (ZnO), compounds of lanthanum-iron (LaFeO3). The type gas VOC detectable by the sensors can be benzene, toluene, one iodo-nonane, decanal and many others.

In a preferred embodiment, an array of sensors is provided, each different from the other solely in the composition of the sensitive or detection material. These are semiconductor chemoresistive sensors apt to disclose gas.

The specificity of the sensors is given by the choice of the combination of specific materials that allow a high degree of discernment between faeces of healthy subjects and faeces of subjects suffering from adenoma or carcinoma of the colon-rectum. The choice of the working temperature and of the actual characteristics of the materials are two fundamental variables that make the array specific for screening by analysis of stool samples.

The materials of the sensitive film of the sensors used are nanostructured semiconductors oxides and the array of sensors present in the instrument is suitable to recognize the gases of interest for the application.

The device 1 also comprises means for feeding the already mentioned flow of air on the faeces sample and sensors or transducers 3.
These feeding means are denoted with a total of 4 and mainly include a pneumatic circuit 41 supplied by a pump, compressor or similar means 40. The circuit 41 pneumatically connects precisely the pump 40 with the environment of the container 6 and with the chambers 11-14 receiving the sensors or transducers 3. In particular, the chambers 11-14 are arranged in series to the environment of the container 6 with respect to the flow of air fed into a main branch 411 of the circuit. There is also provided a secondary branch 412 which realizes a bypass of the feces sample, by connecting directly chambers 11-14 to the pump 40.
On the circuit 41 are further provided adjusting and/or interdiction means of the flow, configured precisely to allow a selective supply of the air flow to the faeces sample and/or to the sensors or transducers 3. In the present example, said means comprises:
- a first flow regulator 45, disposed on the main branch 411 of the circuit 41 upstream of the container 6;
- a second flow regulator 46, placed on the secondary branch 412 of the circuit 41 upstream of the chambers 11-14;
- a first valve 47, arranged on the main branch 411 of the circuit 41 upstream of the regulator 45;
- a second valve 48, arranged on the main branch 411 of the circuit 41 downstream of the container 6.
The flow regulators 45 and 46 may each be automatic or manual.
The valves 47 and 48 are preferably of the on/off type.
Therefore, the means 4 for feeding the flow of air conveys the gas fumes coming from the faeces sample on the sensors/transducers 3 in laminar flow and preferably constant flow. In particular, the pump 40 draws ambient air that is sent to the two flow regulators 45 and 46. The latter flow regulators 45 and 46 allow to modulate the flow so that the same total flow always remains constant during the measurement process. In the main branch 411 of the circuit 41 the valves 47 and 48 are such that air is passed into the container 6 once the instrument is ready for testing. At this point the gaseous mixture of ambient air and faecal exhalations is conveyed to the array of sensors 3 which provide their response to the mixture.

The device 1 also comprises a processing and control unit 5, in communication with the sensors or transducers 3 and preferably with the feeding means 4. The unit 5 is configured to collect and process data from the sensors or transducers 3 and to provide an output datum relating to the presence of cancer or adenoma of the colon-rectum. In the present example, the unit 5 is also configured for the control of the means 4 and in particular of the pump 40 and of the means of adjustment/interdiction of the flow or part of the latter means.
The combination of the responses of the individual sensors is analyzed by an algorithm that provides the negative or positive response of the diagnosis.
The device 1 can also comprise a control and/or communication user interface, such as video.

In a possible embodiment, the algorithm used provides that there is an initial analysis of the stability in air of the electric signal of the sensor (or sensors) which shall not vary by more than 100 mV every 30 minutes. Following stabilization the analysis procedure can be started, after reporting, in particular by video, the program implemented by the instrument. At that point, the operator can open the valves 47 and 48 that allow the pneumatic circuit 41 to pass the air also inside the container with faeces and the duration of the analysis will last until full stabilization of the new value of the electric signal of the sensor (or sensors). At this point the new video signal will suggest the operator to close the valves earlier opened. Differential analysis between the sensor signal in air and sensor signal in the air flow from the sample will be automatic by means of a logic of "yes/no" type based on data collected during the calibration of the instrument.
Of course, the management of the pneumatic circuit, in particular the aforesaid opening and closing of valves, can also automatically occur.
The algorithm of response analysis is based on a principle of analysis of the area identified by the values of the responses of each sensor, placed in the vertices of a pentagon. By comparing the same with a value experimentally estimated through laboratory tests, it is possible to establish the negativity or positivity of the test sample.
The combination of different sensors or transducers provided in the device 1 allows to obtain diagnostic reliability, because each sensor element reacts to various types of gaseous molecules. The device 1 thus allows to identify response patterns different for healthy patients or affected by CCR (also in the form of preliminary polyp) patients, constructed starting from the elaboration of the signals from the individual sensors or transducers.
The present invention has hereto been described with reference to preferred embodiments. It is to be understood that there may be other embodiments within the scope of protection as defined by the claims set out below.

## Claims

1. A detection device (1) for detecting volatile organic compounds in faecal gases, which device is suitable for screening of colorectal cancer and/or colorectal adenoma and comprises:
- a reception seat (2) for a faeces sample;
- a plurality of semiconductor sensors or transducers (3), each configured for detecting one or more respective volatile organic compounds of faecal gases and each of chemoresistive type;
- an outer casing (10) which houses internally said sensors or transducers (3), said outer casing (10) comprising a plurality of chambers (11-14), each housing a respective sensor or transducer or group of sensors or transducers;
- feeding means for supplying a flow of air (4) on the faeces sample and to said sensors or transducers (3),
wherein said feeding means of a flow of air (4) comprises:
▪ a pump (40) configured to draw a flow of ambient air;
▪ a pneumatic circuit (41) connected to said pump (40) and configured to supply said flow of ambient air, in series, on the faeces sample and to said plurality of chambers (11-14);
▪ means for adjusting and/or interdicting the flow of ambient air (45, 46, 47, 48), arranged on said pneumatic circuit (41) and configured in such a way as to allow a selective feeding of the air flow through the faeces sample and/or to said sensors or transducers (3);
▪ bypass means (412, 46) of the faeces sample, that can be activated to supply the flow of ambient air only to said sensors or transducers (3); and
- a processing unit (5), in communication with said sensors or transducers (3) and configured to provide an output datum on the presence of colorectal cancer and/or colorectal adenoma, wherein the arrangement is such that said chambers (11-14), and then the sensors or transducers housed therein, are traversed in series by said flow of ambient air, and
wherein the feeding means (4) is configured to provide a laminar flow of ambient air to the sensors or transducers (3).

2. The device (1) according to claim 1, wherein said reception seat (2) comprises means for removable connection with a container (6) for the faeces sample, which means for removable connection preferably comprises a pair of flanges or jaws configured to engage the container (6).

3. The device (1) according to claim 1 or 2, wherein each of one or more of said sensors or transducers (3) comprises an alumina substrate coated with a film of chemoresistive material.

4. An apparatus for detecting volatile organic compounds in faecal gases, which apparatus is configured for screening of colorectal cancer and/or colorectal adenoma and comprises:
- a device (1) according to any one of the preceding claims; and
- a container for the faeces sample (6) received or receivable in said reception seat (2), wherein preferably said container (6) is of a disposable type.

5. A method for screening of colorectal cancer and/or colorectal adenoma, based on detecting volatile organic compounds in faecal gases, said method employing a device (1) according to any of claims 1 to 3 or an apparatus according to claim 4.

## Patentansprüche

1. Erfassungsgerät (1) zum Erfassen flüchtiger organischer Verbindungen in Fäkalgasen, wobei das Gerät geeignet ist, kolorektalen Krebs und/oder ein kolorektales Adenom zu screenen, und umfasst:
- einen Aufnahmesitz (2) für eine Fäkalienprobe;
- eine Vielzahl von Halbleitersensoren oder -transducern (3), wobei jeder konfiguriert ist, um eine oder mehrere jeweilige flüchtige organische Verbindungen von Fäkalgasen zu erfassen und jeder von einem chemoresistiven Typ ist;
- ein äußeres Gehäuse (10), welches innen die Sensoren und Transducer (3) beherbergt, wobei das äußere Gehäuse (10) eine Vielzahl von Kammern (11-14) umfasst, wobei jede einen jeweiligen Sensor oder Transducer oder eine Gruppe von Sensoren oder Transducern beherbergt;
- Zuführmittel zum Zuführen eines Luftstroms (4) auf die Fäkalienprobe und zu den Sensoren oder Transducern (3), wobei die Zuführmittel für einen Luftstrom (4) umfassen:
▪ eine Pumpe (40), die konfiguriert ist, um einen Umgebungsluftstrom anzusaugen;
▪ eine pneumatische Schaltung (41), die mit der Pumpe (40) verbunden und konfiguriert ist, um den Umgebungsluftstrom nacheinander der Fäkalienprobe und der Vielzahl von Kammern (11-14) zuzuführen;
▪ Mittel zum Einstellen und/oder Verhindern des Umgebungsluftstroms (45, 46, 47, 48), eingerichtet auf der pneumatischen Schaltung (41) und in der Weise konfiguriert, dass eine selektive Zuführung des Luftstroms durch die Fäkalienprobe und/oder zu den Sensoren oder Transducern (3) ermöglicht wird;
▪ Bypass-Mittel (412, 46) der Fäkalienprobe, die aktiviert werden können, um den Umgebungsluftstrom nur den Sensoren oder Transducern (3) zuzuführen; und
- eine Verarbeitungseinheit (5), die sich in Kommunikation mit den Sensoren oder Transducern (3) befindet und konfiguriert ist, um einen Ausgabedatenwert bezüglich der Anwesenheit von kolorektalem Krebs und/oder einem kolorektalen Adenom zur Verfügung zu stellen, wobei die Anordnung derart ist, dass die Kammern (11-14) und dann die Sensoren oder Transducer, die darin beherbergt sind, nacheinander von dem Umgebungsluftstrom durchlaufen werden, und wobei die Zuführmittel (4) konfiguriert sind, um einen laminaren Umgebungsluftstrom zu den Sensoren oder Transducern (3) zu Verfügung zu stellen.

2. Gerät (1) nach Anspruch 1, wobei der Aufnahmesitz (2) Mittel für eine entfernbare Verbindung mit einem Behälter (6) für die Fäkalienprobe umfasst, wobei die Mittel zur entfernbaren Verbindung vorzugsweise ein Paar von Flanschen oder Klauen umfassen, die konfiguriert sind, um den Behälter (6) anzugreifen.

3. Gerät (1) nach Anspruch 1 oder 2, wobei jeder von dem einen oder den mehreren der Sensoren oder Transducer (3) ein Aluminiumsubstrat umfasst, das mit einem Film aus chemoresistivem Material beschichtet ist.

4. Vorrichtung zum Erfassen flüchtiger organischer Verbindungen in Fäkalgasen, wobei die Vorrichtung konfiguriert ist, um kolorektalen Krebs und/oder ein kolorektales Adenom zu screenen, und umfasst:
- ein Gerät (1) nach einem der vorhergehenden Ansprüche; und
- einen Behälter für die Fäkalienprobe (6), die in dem Aufnahmesitz (2) aufgenommen oder aufnehmbar ist, wobei der Behälter (6) zum Wegwerfen ist.

5. Verfahren zum Screenen von kolorektalem Krebs und/oder einem kolorektalen Adenom basierend auf einem Erfassen flüchtiger organischer Verbindungen in Fäkalgasen, wobei das Verfahren ein Gerät (1) nach einem der Ansprüche 1 bis 3 oder eine Vorrichtung nach Anspruch 4 nutzt.

## Revendications

1. Dispositif de détection (1) pour détecter des composés organiques volatils dans les gaz fécaux, lequel dispositif est approprié pour le dépistage du cancer colorectal et/ou de l'adénome colorectal et comprend :
un siège de réception (2) pour un échantillon de matières fécales ;
une pluralité de capteurs ou transducteurs à semi-conducteur (3), chacun configuré pour détecter un ou plusieurs composés organiques volatils respectifs de gaz fécaux et chacun de type chimiorésistant ;
un boîtier externe (10) qui loge intérieurement lesdits capteurs ou transducteurs (3), ledit boîtier externe (10) comprenant une pluralité de chambres (11 - 14), chacune logeant un capteur ou un transducteur ou groupe de capteurs ou de transducteurs respectif ;
un moyen d'alimentation pour fournir un écoulement d'air (4) sur l'échantillon de matières fécales et sur lesdits capteurs ou transducteurs (3),
dans lequel ledit moyen d'alimentation d'un écoulement d'air (4) comprend :
une pompe (40) configurée pour aspirer un écoulement d'air ambiant ;
un circuit pneumatique (41) raccordé à ladite pompe (40) et configuré pour fournir ledit écoulement d'air ambiant, en série, sur l'échantillon de matières fécales et sur ladite pluralité de chambres (11 - 14) ;
un moyen pour ajuster et/ou interdire l'écoulement d'air ambiant (45, 46, 47, 48) agencé sur ledit circuit pneumatique (41) et configuré afin de permettre une alimentation sélective de l'écoulement d'air sur l'échantillon de matières fécales et/ou sur lesdits capteurs ou transducteurs (3) ;
un moyen de dérivation (412, 46) de l'échantillon de matières fécales, qui peut être activé pour alimenter l'écoulement d'air ambiant uniquement sur lesdits capteurs ou transducteurs (3) ; et
une unité de traitement (5), en communication avec lesdits capteurs ou transducteurs (3) et configurée pour fournir une donnée de résultat sur la présence du cancer colorectal et/ou de l'adénome colorectal,
dans lequel l'agencement est tel que lesdites chambres (11 - 14) et par la suite les capteurs ou les transducteurs logés à l'intérieur de ces dernières, sont traversés, en série, par ledit écoulement d'air ambiant, et
dans lequel le moyen d'alimentation (4) est configuré pour fournir un écoulement laminaire d'air ambiant aux capteurs ou aux transducteurs (3).

2. Dispositif (1) selon la revendication 1, dans lequel ledit siège de réception (2) comprend un moyen pour le raccordement amovible avec un récipient (6) pour l'échantillon de matières fécales, lequel moyen pour le raccordement amovible comprend de préférence une paire de brides ou de mâchoires configurées pour venir en prise avec le récipient (6).

3. Dispositif (1) selon la revendication 1 ou 2, dans lequel chacun des un ou plusieurs desdits capteurs ou transducteurs (3) comprend un substrat en alumine recouvert avec un film de matériau chimiorésistant.

4. Appareil pour détecter des composés organiques volatils dans des gaz fécaux, lequel appareil est configuré pour le dépistage du cancer colorectal et/ou de l'adénome colorectal, et comprend :
un dispositif (1) selon l'une quelconque des revendications précédentes ; et
un récipient pour l'échantillon de matières fécales (6) reçu ou pouvant être reçu dans ledit siège de réception (2),
dans lequel de préférence ledit récipient (6) est du type jetable.

5. Procédé pour le dépistage du cancer colorectal et/ou de l'adénome colorectal, basé sur la détection de composés organiques volatils dans des gaz fécaux, ledit procédé utilisant un dispositif (1) selon l'une quelconque des revendications 1 à 3, ou un appareil selon la revendication 4.
